# EUROPEAN PATENT SPECIFICATION

(11) **EP 2 320 966 B1**
(45) Date of publication and mention of the grant of the patent: **11.09.2013**
(21) Application number: 09799932.0
(22) Date of filing: 22.07.2009
(51) Int. Cl.: A61L 27/38, A61F 2/18

(54) **BIOLOGICAL NASAL BRIDGE IMPLANT AND METHOD OF MANUFACTURE**
BIOLOGISCHES NASENRÜCKENIMPLANTAT UND HERSTELLUNGSVERFAHREN
IMPLANT BIOLOGIQUE D'ARCADE NASALE ET PROCÉDÉ POUR SA FABRICATION

(30) Priority: 22.07.2008 CN 200810029656
(43) Date of publication of application: 18.05.2011
(73) Proprietor: Grandhope Biotech Co., Ltd., Guangdong 510663 (CN)
(72) Inventor: XU, Guofeng, Guangzhou Guangdong 510663 (CN); XU, Bin, Guangzhou Guangdong 510663 (CN)
(74) Representative: Hryszkiewicz, Danuta
(86) International application number: PCT/CN2009/000818
(87) International publication number: WO 2010/009616

(56) References cited:
- CN-A- 101 128 225
- CN-A- 101 172 165
- CN-A- 101 332 316
- CN-Y- 201 182 778
- CN-Y- 201 271 394
- US-A- 4 994 084
- US-A- 5 133 754
- US-A- 6 106 555
- US-A1- 2006 030 948
- US-A1- 2007 027 542
- US-A1- 2008 171 906
- US-B1- 6 267 786
- DATABASE WPI Week 200953 Thomson Scientific, London, GB; AN 2009-L93710 XP002682103, & CN 201 271 394 Y (GUANGZHOU ZHIGUANG BIOTECHNOLOGY CO LTD) 15 July 2009 (2009-07-15)

## Description

The present invention relates to a medical prosthesis for human implantation, and in particular, to a biological nasal bridge implant used for nasal bridge augmentation surgery.

Nasal augmentation surgery is the most commonly seen surgery in the field of plastic surgery, and the implants that are currently being used to augment the bridge of the nose are all composed of silicone or Teflon. Although these two materials are biologically inert and may peacefully coexist with the human body after implantation, their composition and structure are not at all similar to the human body so they cannot become part of the host tissue. As a result, it is easy for the implants to shift position, abrade and corrode the skin, and for the implant to be detectable through careful observation, among other defects.
US4994084 discloses nasal homograft implants, made from human dura mater, fascia or unspecified collagen matrix tissue.
US2007027542 discloses heterologous artificial ligaments, made from animal ligaments or tendons.
US5133754 discloses nose implants made from silicone rubber.

Thus, there still remains a need for a nasal bridge implant which avoids the drawbacks described above.

In order to accomplish the objects of the present invention, the present invention provides a nasal bridge implant made according to a method that comprises the following steps:

a) collecting animal material from a bovine or porcine source, the animal material being either a tendon or a ligament;

b) removing cells from the animal material;

c) shaping the animal material to provide a desired shape for the nasal bridge implant;

d) crosslinking the animal material;

e) removing antigens from the animal material;

f) subjecting the animal material to an alkaline treatment;

g) coupling into the animal material active substances which are capable of adhering growth factor and stem cell wherein the active substance is a polypeptide or a glycosaminoglycan, and

h) packing the animal material in a container that contains a sterilization solution.

FIG. 1 is a plan view of a nasal bridge implant according to one embodiment of the present invention.

FIG. 2 is a picture of a sample of nasal bridge implant.

The following detailed description is of the best presently contemplated modes of carrying out the invention. This description is not to be taken in a limiting sense, but is made merely for the purpose of illustrating general principles of embodiments of the invention.The scope of the invention is best defined by the appended claims.

The present invention relates to a biological model nasal bridge implant that is processed and formed using animal tendons/ligaments as the raw material. The raw material is first purified and processed, the cells are removed, and then the material is fixed using epoxy. Thereafter, multifold antigen removal technology, tissue induction technology and a series of other biochemical technological processes are applied. In composition and construction, the nasal bridge implant of the present invention is similar to human tissue, has good biocompatibility and high stability, is not easily degraded, is passively degraded only when the host tissue starts growing in, and does not initiate an immunological rejection response. The implant of the present invention is also able to induce tissue regeneration, to grow together with the host tissue, and to gradually convert itself into host tissue. The implant feels real, and will not cause position shifting, skin abrasion and corrosion.

The present invention provides a preparation method for a biological nasal bridge implant, the nasal bridge implant being formed using animal (preferably bovine or porcine) tendon/ligament as the raw material. The steps of preprocessing, cell removal, shaping, crosslinking and fixation, multifold antigen removal, Alkaline treatment, surface modification with active layer to induce activity, and radiation sterilization, are then applied to the raw material. The specific technical workflow process for preparation is as follows:

1. Preprocessing of animal tendon/ligament

2. Cell removal

3. Shaping/formation

4. Crosslinking

5. Antigen removal

6. Alkaline treatment

7. Surface modification with active layer to induce activity

8. Packaging and Sterilization

Step 1: In step 1 above, animal tendons/ligaments whose basic ingredient is collagen fiber are collected. Preferably, the tendons/ligaments are collected from bovine or porcine sources using techniques that are well-known in the art. Wide-spectrum disinfectants are used to saturate and disinfect the raw material, excess tissue and foreign substances are removed, and then the material is trimmed into a desired size/length that can be further shaped at step 3 below.

Step 2: In the cell removal step, an enzymatic or detergent (surfactant) elution method is used to remove all types of cells from the raw material (tendon or ligament). The enzymes, which can be trypsin and/or pepsin, are used for the enzymolysis of cell. The surfactants, which can be Triton X100, Tween-20, or emulsifier OP-10, are used to breakdown and wash off cell walls.

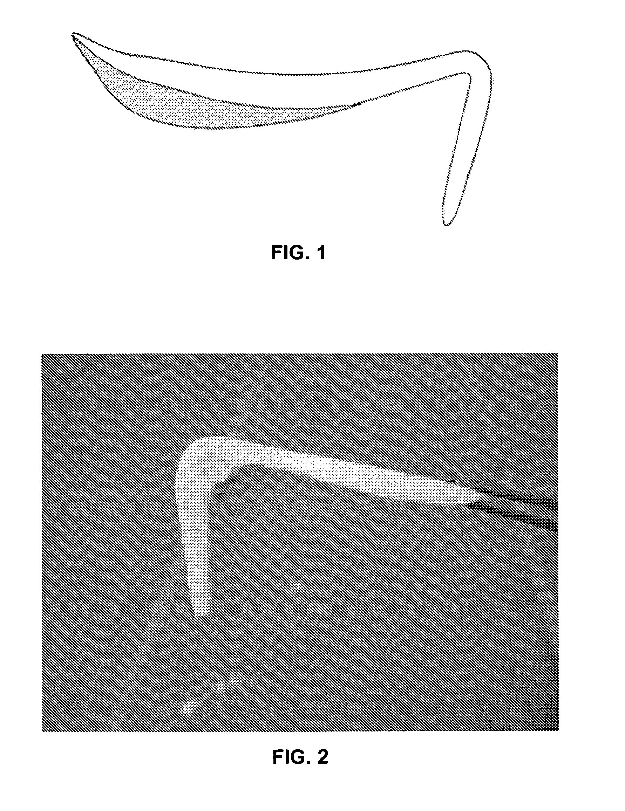

Step 3: In the shaping/formation step, the desired shape for the nasal bridge prosthetic, such as that shown in Figure 1, is formed by further processing that is well-known in the art.

Step 4: The crosslinking and fixation step includes using a crosslinking fixative that uses collagen proteins for crosslinking, which makes the raw material stable. The crosslinking fixative that is used can be the epoxy compound having the following formula: 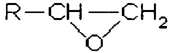 where R = CₙH₂ₙ₊₁ group or 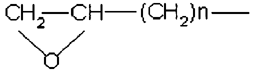 n = 0, 1, 2, 3 ... 12.
The reagent concentration is 0.1-1 N. The reaction temperature is selected between 0-45°C (preferably no higher than 50°C), and the reaction time may be selected between 2 and 96 hours.

Step 5: According to modern immunological theory, the antigenicity of animal tissues stems mainly from active groups located at specific sites and in specific conformations, and these active groups include H₂*,-OH*, -SH*, etc. The specific conformations result mainly from some specific hydrogen bonding formed by spiral protein chains. The specific sites and conformations are called antigen determinants. The antigen removal step uses multiple reagents to block the active groups and alter the special conformation. The reagents used to block specific active groups are mainly nucleophilic reagents that react easily with H₂*,-OH*, -SH* and other similar groups. These reagents include carboxylic acid anhydrides, acyl chlorides, acylamides, epoxy compounds, etc. The reagents that can be used to alter specific conformations include class one strong hydrogen bond formation agents, such as guanidine hydrochloride. Because the specific conformations result mainly from some specific hydrogen bonding formed by spiral protein chains, using strong hydrogen bond formation agents to replace the specific hydrogen bond makes it possible to change the specific conformation. Here the * symbol on the groups indicates that they are a small number of specific groups which are located in specific locations and are able to produce a response to immune signals, and they are not the standard -NH₂, -OH, -SH groups. These specific groups are in a high-energy activity state, preferable for nucleophilic reagent initiated reactions, just as the catalyst's active center is preferable for the reactant or toxin reaction.

Step 6: The alkaline treatment is mainly designed for destroying possibly existing prions. For example, 1-4N sodium hydroxide solution can be used to immerse the prosthesis for 60 minutes at a temperature of 35 ± 2°C. Such processing has already been proven in numerous studies to be effective in destroying prions.

Step 7: In step 7 above, the surface modification includes a process of coupling active substances which are capable of adhering growth factor and stem cell into the prosthesis material, so the prosthesis can adhere and enrich growth factor and stem cell released from human body's self-repair mechanism after implantation, thereby promoting growth factor and stem cell for highly effective expression in the prosthesis over a long period of time, and inducing the stem cells to differentiate into repair tissue mother cells, to again divide and proliferate, regenerate new tissue, and ultimately become autologous nasal bridge tissue. The active substances introduced is a specific polypeptide or glycosaminoglycan compound. Here the specific polypeptides are mainly formed of 16 lysine oligopeptides with arginine, glycine, aspartic acid and other components, for example, a polypeptide constructed of lysine (16) - glycine-arginine-glycine-aspartic acid-serine-proline-cysteine, with the glycosaminoglycan compound being mainly hyaluronic acid, chondroitin sulfate, cortisone sulfate, keratin sulfate, heparin, heparin sulfate, etc. The method of introduction may be accomplished by coupling, chemical adsorption, physical adsorption, or collagen film encapsulation. Coupling is preferred, and coupling agents that may be used are internal carboxylic diacid anhydrides, diacyl dichlorides, diacyl diamides, carbodiimides, and diepoxides.

Step 8: In the packaging and sterilization step, the prosthesis is sealed in a dual-layer plastic bag containing physiological saline storage solution. The packed product is then sterilized under minimum 25 kGy γ-irradiation. This sterilization method has been proven to kill known pathogens, except prions.

Compared to the conventionally-available silicone and Teflon nasal bridge implants, the advantages of the present invention for a biological model nasal bridge implant lie in the fact that it is produced from a purely natural material, its composition is basically similar to that of human tissue, it possesses good biocompatibility, and has no immunological rejection response. After implantation, the implant of the present invention can induce the host tissue to grow into the implant and to heal with the host tissue into one piece, it feels real, there are no irritating foreign materials, and it cannot shift position, corrode or be exposed to the outside, or suffer other complications.

### Example

Obtain fresh and healthy animal tendon/ligament, place in 0.1 % benzalkonium bromide sterilization fluid and saturate for 60 min, then remove foreign substances, repair and trim into the desired size and length. Thereafter, remove, clean, place in trypsin-Tris hydrochloride buffer at room temperature to perform enzymolysis for 2-24 hours. Thereafter, remove, rinse with water, place in a 1% OP-10 solution containing 1 µM benzyl fluorosulfide protease inhibitor, saturate for 8 hours, and remove again. While stirring, use water to rinse three times, remove again, leach out the water content, and create the desired structural shapes and forms such as shown in FIG. 1. Place in a fixation reactor and use a crosslinking agent to perform crosslinking fixation, with the crosslinking agent selected from the epoxy compound described above, or adipoyl chloride with concentration between 0.1-1 N, and react at room temperature for 2-96 hours. After the crosslinking reaction is complete, remove, clean, place in the antigen reactor, add one of the above-described nucleophilic reagents, and react at room temperature for 10-16 hours. Select two different types of reagents and react twice. Then use guanidine hydrochloride solution to react once at a temperature between 5-30°C with a reaction time of 8-24 hours. Remove, clean, place in 1-24 sodium hydroxide solution at 30-35°C, saturate and process for 60 minutes, and then discard the reaction fluid. Use diluted acid to neutralize the residual sodium hydroxide, and then clean. Place in the special-use reactor for surface modification, add the lysine (16) - glycine-arginine-glycine-aspartic acid-serine-proline-cysteine polypeptide and the adipoyl chloride coupling reagent, then react in moderate conditions for 8-16 hours at 25 ± 2°C. Remove, and wash thoroughly. Seal using physiological saline storage solution in a dual-layer plastic bag, send for radiation sterilization, and obtain the finished product.

## Claims

1. A method of preparing a nasal bridge implant, comprising the steps of:
a) collecting an animal material from a bovine or porcine source, the animal material being either a tendon or a ligament;
b) removing cells of the animal material;
c) shaping the animal material into a desired shape for the nasal bridge implant;
d) crosslinking the animal material;
e) removing antigens of the animal material;
f) treating the animal material by using an alkaline solution;
g) coupling into the animal material an active substance capable of adhering growth factors and stem cells, wherein the active substance is a polypeptide or a glycosaminoglycan; and
h) packing the animal material in a container that contains a sterilization solution.

2. The method of claim 1, **characterized in that** in the step b), the cells are removed by using an enzymatic method or a detergent elution method.

3. The method of claim 2, **characterized in that** an enzyme used in the enzymatic method is trypsin or pepsin.

4. The method of claim 2, **characterized in that** a detergent used in the detergent elution method is Triton X100, Tween-20, or emulsifier OP-1 0.

5. The method of claim 1, **characterized in that** in the step d), a crosslinking agent involved therein is an epoxy compound represented by a formula of 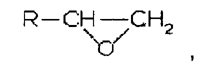 wherein R = CₙH₂ₙ₊₁ group, or by a formula of 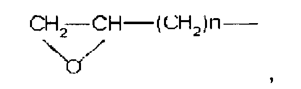 wherein n = 0, 1, 2, 3, ..., 12.

6. The method of claim 1, **characterized in that** in the step e), a nucleophilic reagent and a strong hydrogen bond formation agent that easily react with hydrogen of -NH₂, -OH, -SH, and other groups are introduced to block specific groups and to change specific conformations of the animal material.

7. The method of claim 6, **characterized in that** the nucleophilic reagent is a carboxylic acid anhydride, an acyl chloride, an acylamide, or an epoxide, and the strong hydrogen bonding agent is a guanidine compound.

8. The method of claim 1, **characterized in that** in the step f), the alkaline solution is 1-4N sodium hydroxide solution and the animal material is immersed therein.

9. The method of claim 1, **characterized in that** the polypeptide is lysine (16)-glycine-arginine-glycine-aspartic acid-serine-proline-cysteine.

10. A nasal bridge implant made according to a method comprising the steps of:
a) collecting an animal material from a bovine or porcine source, the animal material being either a tendon or a ligament;
b) removing cells of the animal material;
c) shaping the animal material into a desired shape for the nasal bridge implant;
d) crosslinking the animal material;
e) removing antigens of the animal material;
f) treating the animal material by using an alkaline solution;
g) coupling into the animal material an active substance capable of adhering growth factors and stem cells, wherein the active substance is a polypeptide or a glycosaminoglycan; and
h) packing the animal material in a container that contains a sterilization solution.

11. The implant of claim 10, **characterized in that** in the step b), the cells are removed by using an enzymatic method or a detergent elution method.

12. The implant of claim 10, **characterized in that** a crosslinking agent involved therein is an epoxy compound represented by a formula of 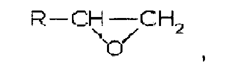 R = CₙH₂ₙ₊₁ group, or by a formula of 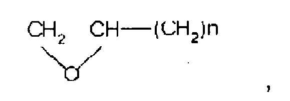 wherein n = 0, 1, 2, 3, ..., 12.

13. The implant of claim 10, **characterized in that** a nucleophilic reagent and a strong hydrogen bond formation agent that easily react with hydrogen of -NH₂, -OH, -SH, and other groups are used to block specific groups and to change specific conformations of the animal material, the nucleophilic reagent being a carboxylic acid anhydride, an acyl chloride, an acylamide, or an epoxide, and the strong hydrogen bonding agent being a guanidine compound.

14. The implant of claim 10, **characterized in that** in the step f), the alkaline solution is 1-4N sodium hydroxide solution and the animal material is immersed therein.

15. The implant of claim 10, **characterized in that** the polypeptide is lysine (16)-glycine-arginine-glycine-aspartic acid-serine-proline-cysteine.

## Patentansprüche

1. Verfahren des Herstellens eines Nasenrückenimplantats, umfassend die folgenden Schritte:
a) Entnehmen eines tierischen Materials aus einer bovinen oder porzinen Quelle, wobei es sich bei dem tierischen Material um eine Sehne oder ein Band handelt;
b) Entfernen von Zellen des tierischen Materials;
c) Formen des tierischen Materials zu einer gewünschten Form für das Nasenrückenimplantat;
d) Vernetzen des tierischen Materials;
e) Entfernen von Antigenen des tierischen Materials;
f) Behandeln des tierischen Materials durch Verwendung einer alkalischen Lösung;
g) Koppeln einer aktiven Substanz in das tierische Material, die zum Anlagern von Wachstumsfaktoren und Stammzellen in der Lage ist, wobei es sich bei der aktiven Substanz um ein Polypeptid oder ein Glycosaminoglycan handelt; und
h) Packen des tierischen Materials in einen Behälter, der eine Sterilisierungslösung enthält.

2. Verfahren nach Anspruch 1, **dadurch gekennzeichnet, dass** die Zellen in Schritt b) durch Anwendung eines enzymatischen Verfahrens oder eines Detergenselutionsverfahrens entfernt werden.

3. Verfahren nach Anspruch 2, **dadurch gekennzeichnet, dass** es sich bei einem bei dem enzymatischen Verfahren verwendeten Enzym um Trypsin oder Pepsin handelt.

4. Verfahren nach Anspruch 2, **dadurch gekennzeichnet, dass** es sich bei einem in dem Detergenselutionsverfahren verwendeten Detergens um Triton X100, Tween-20 oder Emulgator OP-1 0 handelt.

5. Verfahren nach Anspruch 1, **dadurch gekennzeichnet, dass** es sich in Schritt d) bei dem daran beteiligten Vernetzungsmittel um eine Epoxidverbindung handelt, die durch eine Formel 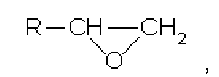 wobei R für eine CₙH₂ₙ₊₁ -Gruppe steht, oder eine Formel 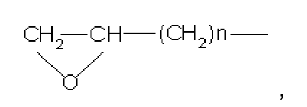 wobei n für 0, 1, 2, 3, ..., 12 steht, wiedergegeben ist.

6. Verfahren nach Anspruch 1, **dadurch gekennzeichnet, dass** in Schritt e) ein nukleophiles Reagens und ein starkes Wasserstoffbindungsbildungsmittel, das leicht mit Wasserstoff von -NH₂, -OH, -SH reagiert, und andere Gruppen eingeführt werden, um spezifische Gruppen zu blockieren und um spezifische Konformationen des tierischen Materials zu verändern.

7. Verfahren nach Anspruch 6, **dadurch gekennzeichnet, dass** es sich bei dem nukleophilen Reagens um ein Carbonsäureanhydrid, ein Acylchlorid, ein Acylamid oder ein Epoxid handelt und es sich bei dem starken Wasserstoffbindungsmittel um eine Guanidinverbindung handelt.

8. Verfahren nach Anspruch 1, **dadurch gekennzeichnet, dass** es sich bei der alkalischen Lösung in Schritt f) um 1-4 N Natriumhydroxidlösung handelt und das tierische Material darin untergetaucht ist.

9. Verfahren nach Anspruch 1, **dadurch gekennzeichnet, dass** es sich bei dem Polypeptid um Lysin(16)-Glycin-Arginin-Glycin-Asparaginsäure-Serin-Prolin-Cystein handelt.

10. Nasenrückenimplantat, das nach einem Verfahren hergestellt ist, welches folgende Schritte umfasst:
a) Entnehmen eines tierischen Materials aus einer bovinen oder porzinen Quelle, wobei es sich bei dem tierischen Material um eine Sehne oder ein Band handelt;
b) Entfernen von Zellen des tierischen Materials;
c) Formen des tierischen Materials zu einer gewünschten Form für das Nasenrückenimplantat;
d) Vernetzen des tierischen Materials;
e) Entfernen von Antigenen des tierischen Materials;
f) Behandeln des tierischen Materials durch Verwendung einer alkalischen Lösung;
g) Koppeln einer aktiven Substanz in das tierische Material, die zum Anlagern von Wachstumsfaktoren und Stammzellen in der Lage ist, wobei es sich bei der aktiven Substanz um ein Polypeptid oder ein Glycosaminoglycan handelt; und
h) Packen des tierischen Materials in einen Behälter, der eine Sterilisierungslösung enthält.

11. Implantat nach Anspruch 10, **dadurch gekennzeichnet, dass** die Zellen in Schritt b) durch Anwendung eines enzymatischen Verfahrens oder eines Detergenselutionsverfahrens entfernt werden.

12. Implantat nach Anspruch 10, **dadurch gekennzeichnet, dass** es sich bei einem daran beteiligten Vernetzungsmittel um eine Epoxidverbindung handelt, die durch eine Formel 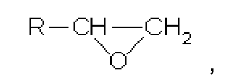 wobei R für eine CₙH₂ₙ₊₁ -Gruppe steht, oder eine Formel 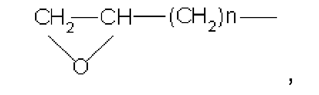 wobei n für 0, 1, 2, 3, ..., 12 steht, wiedergegeben ist.

13. Implantat nach Anspruch 10, **dadurch gekennzeichnet, dass** ein nukleophiles Reagens und ein starkes Wasserstoffbindungsbildungsmittel, das leicht mit Wasserstoff von -NH₂, -OH, -SH reagiert, und andere Gruppen verwendet werden, um spezifische Gruppen zu blockieren und um spezifische Konformationen des tierischen Materials zu verändern, wobei es sich bei dem nukleophilen Reagens um ein Carbonsäureanhydrid, ein Acylchlorid, ein Acylamid oder ein Epoxid handelt und es sich bei dem starken Wasserstoffbindungsmittel um eine Guanidinverbindung handelt.

14. Implantat nach Anspruch 10, **dadurch gekennzeichnet, dass** es sich bei der alkalischen Lösung in Schritt f) um 1-4 N Natriumhydroxidlösung handelt und das tierische Material darin untergetaucht ist.

15. Implantat nach Anspruch 10, **dadurch gekennzeichnet, dass** es sich bei dem Polypeptid um Lysin(16)-Glycin-Arginin-Glycin-Asparaginsäure-Serin-Prolin-Cystein handelt.

## Revendications

1. Procédé de préparation d'un implant d'arcade nasale, ledit procédé comprenant les étapes de :
a) collecte d'un matériau animal d'une source bovine ou porcine, le matériau animal étant soit un tendon soit un ligament ;
b) élimination des cellules du matériau animal ;
c) façonnage du matériau animal en une forme souhaitée pour l'implant d'arcade nasale ;
d) réticulation du matériau animal ;
e) élimination des antigènes du matériau animal ;
f) traitement du matériau animal en utilisant une solution alcaline ;
g) couplage du matériau animal à une substance active capable d'adhérer aux facteurs de croissance et aux cellules souches, dans lequel la substance active est un polypeptide ou un glycosaminoglycane ; et
h) conditionnement du matériau animal dans un récipient qui contient une solution de stérilisation.

2. Procédé selon la revendication 1, **caractérisé en ce que** dans l'étape b), les cellules sont éliminées en utilisant un procédé enzymatique ou un procédé d'élution avec un détergent.

3. Procédé selon la revendication 2, **caractérisé en ce qu'**une enzyme utilisée dans le procédé enzymatique est la trypsine ou la pepsine.

4. Procédé selon la revendication 2, caractérisé en qu'un détergent utilisé dans le procédé d'élution avec un détergent est le Triton X100, le Tween-20, ou l'émulsifiant OP-10.

5. Procédé selon la revendication 1, **caractérisé en ce que** dans l'étape d), un agent de réticulation qui est impliqué est un composé époxy représenté par la formule 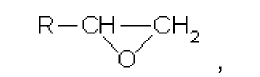 dans laquelle R = un groupe CₙH₂ₙ₊₁, ou par la formule 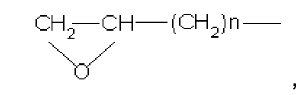 dans laquelle n = 0, 1, 2, 3, ..., 12.

6. Procédé selon la revendication 1, **caractérisé en ce que** dans l'étape e), un réactif nucléophile et un agent de formation de liaisons hydrogène fortes qui réagissent facilement avec l'hydrogène de -NH₂, -OH, -SH, et d'autres groupes sont introduits pour bloquer des groupes spécifiques et pour modifier des conformations spécifiques du matériau animal.

7. Procédé selon la revendication 6, **caractérisé en ce que** le réactif nucléophile est un anhydride d'acide carboxylique, un chlorure d'acyle, un acylamide, ou un époxyde, et l'agent de formation de liaisons hydrogène fortes est un composé guanidine.

8. Procédé selon la revendication 1, **caractérisé en ce que** dans l'étape f), la solution alcaline est une solution d'hydroxyde de sodium 1 à 4 N et le matériau animal est immergé dans ladite solution.

9. Procédé selon la revendication 1, **caractérisé en ce que** le polypeptide est lysine (16)-glycine-arginine-glycine-acide aspartique-sérine-proline-cystéine.

10. Implant d'arcade nasale fabriqué selon un procédé comprenant les étapes de :
a) collecte d'un matériau animal d'une source bovine ou porcine, le matériau animal étant soit un tendon soit un ligament ;
b) élimination des cellules du matériau animal ;
c) façonnage du matériau animal en une forme souhaitée pour l'implant d'arcade nasale ;
d) réticulation du matériau animal ;
e) élimination des antigènes du matériau animal ;
f) traitement du matériau animal en utilisant une solution alcaline ;
g) couplage du matériau animal à une substance active capable d'adhérer aux facteurs de croissance et aux cellules souches, dans lequel la substance active est un polypeptide ou un glycosaminoglycane ; et
h) conditionnement du matériau animal dans un récipient qui contient une solution de stérilisation.

11. Implant selon la revendication 10, **caractérisé en ce que** dans l'étape b), les cellules sont éliminées en utilisant un procédé enzymatique ou un procédé d'élution avec un détergent.

12. Implant selon la revendication 10, **caractérisé en ce qu'**un agent de réticulation impliqué est un composé époxy représenté par la formule 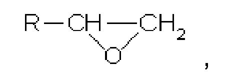 R = un groupe CₙH₂ₙ₊₁, ou par la formule 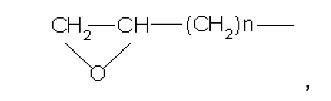 dans laquelle n = 0, 1, 2, 3, ..., 12.

13. Implant selon la revendication 10, **caractérisé en ce qu'**un réactif nucléophile et un agent de formation de liaisons hydrogène fortes qui réagissent facilement avec l'hydrogène de -NH₂, -OH, -SH, et d'autres groupes sont utilisés pour bloquer des groupes spécifiques et pour modifier des conformations spécifiques du matériau animal, le réactif nucléophile étant un anhydride d'acide carboxylique, un chlorure d'acyle, un acylamide, ou un époxyde, et l'agent de formation de liaisons hydrogène fortes étant un composé guanidine.

14. Implant selon la revendication 10, **caractérisé en ce que** dans l'étape f), la solution alcaline est une solution d'hydroxyde de sodium 1 à 4 N et le matériau animal est immergé dans ladite solution.

15. Implant selon la revendication 10, **caractérisé en ce que** le polypeptide est lysine (16)-glycine-arginine-glycine-acide aspartique-sérine-proline-cystéine.
